# EUROPEAN PATENT APPLICATION

(11) **EP 1 671 656 A1**
(43) Date of publication of application: **21.06.2006**
(21) Application number: 05257679.0
(22) Date of filing: 14.12.2005
(51) Int. Cl.: A61K 49/22

(54) **Gels composed of glycols and/or polyols and PVP as in-vivo biocompatible acoustic couplants**

(30) Priority: 14.12.2004 US 635845 P; 19.10.2005 US 253320
(71) Applicant: Sonotech, Inc., Bellingham, WA 98225 (US)
(72) Inventor: Smith, Larry L., Lummi Island, Washington 98262 (US)
(74) Representative: Gill, Stephen Charles

(57) **Abstract**

A waterless in vivo biocompatible and bio-excretable lubricant and ultrasound coupling fluid or gel produced from formulations based on polyvinylpyrrolidone (PVP) and one or more glycols and/or polyols such as propylene glycol, glycerol, polyethylene glycol and sorbitol.

## Description

The present invention is directed toward the medical use of polyvinypyrrolidone (PVP) as an in vivo biocompatible acoustic coupling gel and instrument lubricant for use in ultrasound imaging, doppler based flow measurement, and High Intensity Focused Ultrasound (HIFU) therapy when the gel may be transported inside the body, such as during surgery and with invasive procedures.

Medical applications of ultrasound generally utilize electromagnetic wave frequencies that typically range between 0.5 and 20 MHz for imaging, High Intensity Focused Ultrasound (HIFU) therapy and flow measurements. Ultrasound energy at these frequencies is poorly transmitted by air, which therefore, requires a coupling or conduction medium that possesses acoustic properties similar to tissue and organs. Such media can consist of fluids, gels and certain solid materials and films, to transfer the acoustic energy between the body and the electronics of the diagnostic instrument. This media is commonly referred to as an ultrasound couplant, ultrasound gel, ultrasound transmission media or acoustic transmission media. Many fluids and water-based gels have been used as ultrasound couplants over the years. Early use of mineral oil was replaced by gels of water and acrylic based polymers such as CARBOPOL™ (a trademark of BF Goodrich Specialty Chemicals) typical of those described in U.S. Patent No. 4,002,221 to Buchalter, and also gels made from acrylic polymers and attached as coupling members to transducers such as are described in U.S. Patent No. 4,459,854 to Richardson et al. as a method for improvement of peri-vascular blood flow measurement.

The above materials and methods to transfer and couple ultrasound energy between the active face of an ultrasound transducer or suitable acoustic standoff or delay line and the human or animal body (i.e. such ultrasound coupling fluids and gels when used in surgical, and ultrasound guided needle puncture procedures) have fundamental disadvantages that place the patient at risk. Some of these disadvantages are described below:

1. Oils or thickened water-based gels typically used in medical ultrasound are similarly described as in previously discussed U.S. Patent No. 4,002,221, and are comprised of compounds such as acrylic polymers, carboxy alkyl cellulose, hydroxyethylcellulose, carboxy polymethylene, polyalkylene glycol humectants, organic acids, alkali metal salts, parabens and other germicidal and fungicidal agents, and surfactants that are unsuitable for use in applications where they may be carried into the body tissue or fluids.

2. The above-mentioned couplants are also commercially available in sterilized form, thus implying and encouraging their inappropriate use in vivo, where their chemical constituents are known to either be harmful to the human body or have not been evaluated for in vivo use.

3. Currently available ultrasound couplants supplied in sterile form contain acrylic polymers such as CARBOPOL as a common and primary ingredient. CARBOPOL, for example, has not been tested for in vivo biocompatibility and is not recommended for such use. Some currently available sterile couplants also contain cellulose ethers to increase salt stability. According to E. Doecker in "Water Swollen Cellulose Derivatives in Pharmacy" from Hydrogels in Medicine and Pharmacy: Vol. 2-Polymers, edited by Peppas N. A., CRC Press Inc., Boca Raton, Florida, 1987, pg. 124, "In common use, such celluloses are used orally and externally, however parenteral administration of cellulose is not recommended since derivatives are not easily metabolized". Since various chemicals in these formulations are not in vivo biocompatible and may not biodegrade, they can remain in the body and cause inflammation, disruption in flow of lymph, irritation, anaphylactic shock and other immune system reactions. This concern becomes apparent during ultrasound guided needle biopsy or aspiration, or when ultrasound transducers are used inside the body for imaging during surgery in contact with organs, tissue and blood.

4. Of additional concern are the unknown chemical constituents formed during sterilization processing. Methods of couplant sterilization include steam autoclave, E-beam, broad spectrum light and gamma radiation protocols. Couplants that incorporate CARBOPOL in the formulation can break down from heating during the autoclave cycle. When exposed to ionizing radiation, such as in the case of gamma, E-beam, and high intensity light sterilization, destructive free radicals can form that initiate chain scission, cross linking and formation of unknown new compounds. This activity is evidenced by presence of bubbles, changes in color, viscosity and mechanical properties of the polymer products after sterilization.

5. It is important to note that sterility of a substance does not guarantee biocompatibility, or of greater importance, in vivo biocompatibility. When a substance is sterile, it does not contain live microorganisms; however, such sterile materials may not be in vivo-biocompatible should they contain compounds that are incompatible with tissue or body fluids. For example, natural and synthetic materials that are recognized by the FDA as GRAS (Generally Regarded As Safe) may not be in vivo biocompatible. An in vivo biocompatible substance is both sterile and contains no living micro-organisms or pyrogens. Presence of chemicals and protein remnants of microorganisms can be toxic to the human body and cause inflammation or immune system reactions. A substance such as the device of this invention is in vivo biocompatible as an ultrasound couplant in contact with human tissue and body fluids and biodegrades thus being eliminated through natural physiological pathways.

U.S. Patent No. 6,302,848 to Larson et al. describes an ultrasound coupling gel that is biocompatible and degradable in vivo, consisting and limited to water, propylene glycol and polyethylene oxide of various molecular weights. The formulations of Larson et al. do not teach the use of PVP as an ultrasound couplant.

It is an object of the present invention to provide ultrasound couplants and device lubricants for use in medical ultrasound applications where such formulations may contact body tissue, fluids and organs and when used as a lubricant to facilitate the passage of imaging devices into body cavities.

It is a further object of the present invention to provide gels and fluids that are biocompatible and degradable in vivo, and appropriate for use in medical diagnostic and therapeutic ultrasound procedures that are invasive to the human body during surgery, guided biopsy, within body cavities and ophthalmic imaging.

The present invention is directed to an in vivo biocompatible and bio-excretable lubricant and ultrasound coupling fluid or gel produced from formulations based on polyvinylpyrrolidone (PVP) that do not contain water. The inventive couplant fluid or gel may be derived from formulations based upon solutions of polyvinylpyrrolidone of various molecular weights, e.g. PVP plasticized in alkylene and polyalkylene glycols, to achieve desired tactile and drying characteristics. Thus, an aspect of the present invention provides an in vivo biocompatible and bio-excretable lubricant and ultrasound couplant fluid or gel consisting of or comprising:
1-99 wt.% glycols, polyols and/or fats and esters thereof, polyvinylpyrrolidone, which may be crosslinked or uncrosslinked,
optional organic polyvinylpyrrolidone cross-linkers selected from at least one of chitosan derivatives, polyethyleneimine, amines and aldehydes, and
optional monoglycerides and/or triglycerides,
said fluid or gel containing no water.

The PVP may be cross-linked. Thus additionally, the inventive fluids and gels may be prepared by addition of organic cross-linkers such as chitosan derivatives, polyethylene imine, and various amines and aldehydes. Such formulations of PVP and blends of PVP are cross-linked to form, for example, adhesive membranes used in burn and wound care. Thus the PVP may be crosslinked by, or the fluid or gel may further comprise, organic cross-linkers selected from at least one of chitosan derivatives, polyethyleneimine, amines and aldehydes.

The hydrophilic polymeric compound, PVP meets the requirements of in vivo biocompatibility and elimination from the body by natural pathways and processes. The gels of the inventive device are formulated with one or more of glycols (which term encompasses polyglycols), polyols, and/or fats and esters thereof in a weight percentage range between 1 and 99 % together with polyvinylpyrrolidone. The weight percentage of PVP practical for production of useful ultrasound couplants can vary with the molecular weight of the PVP and the base solvent in the range of 1.0 % and 60 %. The molecular weight of PVP and the base plasticizer can be selected according to desired viscosity, tactile qualities and imaging application. When prepared in final form, such mixtures can exhibit acoustic properties similar to that of human tissue, render acceptable low levels of artifact, distortion and attenuation of the ultrasound energy, and acceptable viscosity, film forming and adherence characteristics.

The present invention is related to the medical use of acoustic coupling fluids and gels used in vivo ultrasound imaging, Doppler-based flow measurement and in ultrasound guided transcutaneous biopsy and in High Intensity Focused Ultrasound (HIFU) therapy.

The present invention is a medical device lubricant and ultrasound coupling media in gel or liquid form, comprised of polyvinylpyrrolidone (PVP), humectants and plasticizer such as glycols and polyols that act so as to provide acceptable viscosity, tactile qualities and drying rates. Formulations so composed have acceptable long-term interaction in vivo that render the acoustic media biocompatible with human tissue, organs and body fluids.

The inventive lubricant and acoustic coupling fluids or gels comprise polyvinylpyrrolidone with e.g. glycol and polyol plasticizers that specifically do not contain water. The glycols may be polyglycols. Additionally, the formulations of the invention provide ultrasound couplants that typically are not sticky and do not form the cobweb strings that are characteristic of hydrogels produced by polyethylene oxides. The couplants of the device of this invention and are typically smooth non-sticky plasticized thick liquids that are easily applied and removed from patients and instrumentation, yet impart required ultrasound transmission characteristics.

Gels of polyvinylpyrrolidone (PVP) can be prepared as thickened solutions in water or alcohols. However, and in accordance with the present invention, they can also be prepared in solution with plasticizers such as glycols, polyols and/or fats and esters thereof, preferably containing two or greater carbon atoms and more preferably 2 to 6 carbon atoms, in a wt. % range of about 1% to about 99%, preferably about 10% to about 98%, and most preferred 70 to 98%. The preferred glycols comprise propylene glycol, glycerol and polyethylene glycol of various molecular weights. Should a higher alcohol be added to the formulation, sorbitol is preferred. Polyethylene glycol in a blend with propylene glycol is most preferred since the blend is in vivo biocompatible and biodegradable and in the preferred embodiment, functions as humectants to increase drying time, an antimicrobial and freeze inhibitor.

It is well known in the art to crosslink PVP so as to increase viscosity and modify the physical and mechanical properties. Such cross-linking techniques include high-energy radiation such as from e-beam and gamma sources and by chemically cross-linking, for example, with an amine containing polymers such as polyethyleneimine and chitosan. U.S. Patents Nos. 5,306,504; 5,420,197 and 5,645,855 to Lorenz teach methods of cross-linking PVP using poly-functional amines. U.S. Patent No. 6,379,702 to Lorenz et al. teaches production of cross-linked PVP with aqueous solutions of chitosan derivatives. Such cross-linked gels of PVP tend to be adhesive in nature and are commonly used as absorbent wound dressings and sealants.

The adhesivity and rheology of the cross-linked gels of Lorenz restrict free motion of instruments such as an ultrasound probe when in contact with such materials thus limiting acceptability of these formulations for ultrasound couplants or instrument lubricants. By comparison, the inventive gels composed of PVP and humectants formulated as pseudoplastic gels of various viscosities can provide the utility of being spread into thin lubricous films thus facilitating free motion of an ultrasound transducer over the examination site.

PVP is commercially available from BASF Corporation in various grades which differ as to technical or pharmaceutical applications and molecular weight. The BASF designation for pharmaceutical PVP is Kollidon and further described by K-value, which is an indicator of molecular weight. The typical range of molecular weights that can be used in the device of this invention begins at 2000 - 3000 for Kollidon 12 PF, 40,000-54,000 for Kollidon 30 and extends to 1,000,000-1,500,000 daltons for Kollidon 90. The K-value is also indicative of the viscosity of a solution of a given percentage and increases as the K-value increases. The most preferred polymer grade for the device of this invention is Kollidon 30, due to the tactile characteristics of glycol and polyol solutions made therefrom.

Polyvinylpyrrolidone is readily soluble in water and most alkyl alcohols, glycols (including polyglycols) and polyols of which members of these families are known to be in vivo biocompatible and biodegradable. In addition, formulation in the absence of water provides for more efficient viscosity building and reduction in string formation and stickiness, notable in the gels of Larson et al. discussed above. Glycols and polyols were selected as the most desired compounds in the formulation of the inventive device without limiting the use of other compounds in the above-mentioned families of compounds. Of these compounds, polyethylene glycols, glycerol and propylene glycol are preferred.

To determine the viscosity building capacity of PEG, propylene glycol and glycerol, 10 % samples of PVP K-90 in the base solvents were prepared and evaluated.

**Table 1. Effect of Plasticizer on Final Viscosity**

| Formulation | 10% PVP K-90 | 10% PVP K-90 | 10% PVP K-90 |
|---|---|---|---|
| | 90% Propylene Glycol | 90% PEG 300 | 90% Glycerol |
| Viscosity (cps) | 17,760 #3 LVT | 28,040 | 336,000 |
| | @ 1.5 rpm | # 3 LVT @1.5 rpm | # 4 LVT @ 0.6 rpm |

The data in Table 1 demonstrates the viscosity building effects of three plasticizers that consist in the formulations of the device of this invention and indicates the potential to formulate PVP in one or more plasticizers to obtain desired tactile and viscosity characteristics.

Polyvinylpyrrolidone is soluble in water, alcohols, glycols (including polyglycols), polyols, alkyl chlorides and amines in addition to compounds of lesser biocompatibility. In vivo biocompatible and degradable compounds such as glycerol and propylene glycol, and polyglycols, individually or combinations thereof can be formulated with PVP as lubricants and ultrasound couplants. Additionally, monoglycerides and triglycerides such as those sourced from soybean oil can be used to modify the tactile and drying characteristics of couplant formulations. Glycerol and polyethylene glycols are efficient viscosity builders with glycerol being the most efficient.

Formulations of PVP with polyglycols favor PEG 300 and 600 in preference to PEG with higher molecular weights, since these are generally solids at room temperature and tend to precipitate over time at room temperature. In general, as the molecular weight of the PEG increases, the resultant viscosity also increases. Two formulations, of 8.5 % PVP K 90 and 61.5 % propylene glycol were prepared, the first containing 30 % PEG 300 and the second 30 % PEG 600. The data in Table 2 following demonstrates the effect of increasing the molecular weight of PEG on resultant viscosity.

**Table 2. PEG Molecular Weight vs. Viscosity**

| Formulation | 8.5% PVP K-90 | 8.5% PVP K-90 |
|---|---|---|
| | 61.5% Propylene Glycol | 61.5% Propylene Glycol |
| | 30% PEG 300 | 30% PEG 600 |
| Viscosity #2 LVT @ 1.5 rpm | 10,450 cps | 13,800 cps |

Based on the data summarized in Tables 1 and 2, a series of formulations were prepared to evaluate the tactile, viscosity and relative drying characteristics. Formulations of PVP grades K-30 and K-90 were prepared in base solvents of propylene glycol, PEG 300, PEG 600, glycerol and blends thereof and evaluated for production of gels with acceptable viscosity and tactile qualities. Table 3 is a comparative summary of these formulations and resultant viscosity values.

**Table 3. Example Formulations**

| Formula | PVP K 15 | PVP K 30 | PVP K 90 | PEG 300 | PEG 600 | PEG 8000 | Propylene Glycol | Glycerol | Viscosity (CPS) |
|---|---|---|---|---|---|---|---|---|---|
| 1 | 10 | | | | 30 | | 60 | | 400 |
| 2 | | 20 | | | 20 | | 60 | | 940 |
| 3 | | 22 | | | 30 | | 48 | | 3,380 |
| 4 | | | 10 | 90 | | | | | 28,040 |
| 5 | | 5 | | 95 | | | | | 3,890 |
| 6 | | 24 | | | 28 | | 48 | | 3,920 |
| 7 | | 26 | | | 26 | | 48 | | 5,860 |
| 8 | | 10 | | | | | 90 | | 17,760 |
| 9 | | 10 | | 5 | | | 5 | 80 | 5,920 |
| 10 | | 7.5 | | | | | | 92.5 | 4,760 |
| 11 | | 10 | | | | | | 90 | 8,360 |
| 12 | | | 10 | | | | | 90 | 336,000 |
| 13 | | | 5 | | | | | 95 | 51,600 |
| 14 | | | 2.5 | | | | | 97.5 | 15,720 |
| 15 | | | 8 | 30 | | | 62 | | 8,800 |
| 16 | | | 8 | | 30 | | 62 | | 6,080 |
| 17 | | | 8.5 | | 30 | | 61.5 | | 13,800 |
| 18 | | | 8.5 | 30 | | | 61.5 | | 10,450 |
| 19 | | | 9 | | 30 | | 61 | | 14,640 |
| 20 | | | 10 | 30 | | | 60 | | 15,920 |
| 21 | | | 8 | | 30 | | 62 | | 11,100 |

Table 3 provides examples of formulations of glycols and polyglycols with ranges of polymer concentrations that produce in vivo biocompatible and biodegradable ultrasound couplants. Such compositions have mechanical, chemical and acoustic properties suitable for use as instrument lubricants and couplants for ultrasound examinations. It can be appreciated that different combinations of ingredients, and concentrations thereof, can be formulated so as to produce ultrasound couplants with different mechanical, chemical and acoustic properties. In the present invention, use of glycols and polyols in the absence of water produces gels of acceptable viscosity with improved lubricity and drying time as compared to formulations containing water.

In practice, the ideal viscosity and film-forming requirements are determined by the type of ultrasound imaging procedure performed. With exception of gels used for transcutaneous scanning of large areas such as the abdomen, gel viscosities exceeding 5,000 cps are preferred.

The present invention is an in vivo biocompatible and biodegradable lubricant and ultrasound couplant that is formulated without water to be, desirably, a strong and adherent film former, lubricious, and possessing acoustic properties acceptable for transmission of ultrasound in diagnostic imaging procedures. Such example formulations as those shown in Table 3 can be safely used inside the human body during surgery and HIFU procedures and applications where ultrasound couplant can be transferred into the body such as during image guided biopsy and amniocentesis.

In addition to the characteristics noted above, tactile properties are notable requirements for user acceptance. Gels such as the polyethylene oxide based in vivo biocompatible gels of Larson et al., discussed above, are sticky and by nature of the polymer tend to form strings when applied to and removed from instruments and examination areas. Such characteristics limit user acceptance and use. The waterless couplants of this inventive device limit and eliminate such stickiness and string formation providing for ease of use and acceptability.

A preferred formulation of the inventive device as described in Table 3 is Example #3, Example #6 is more preferred and the most preferred is Example #7.

The use of lower molecular weight, Kollidone 30 is advantageous since it is readily eliminated from the body through normal physiological pathways. In comparison, higher molecular weight PVP such as Kollidone 90 is not readily eliminated through the kidneys and is thus retained in the body for longer time periods. Additionally, stickiness and tendency of PVP solutions to form strings decreases as molecular weight is decreased. As the concentration of Kollidone 30 is increased to 30 % and more, the solutions remain lubricious and do not form strings. Example 7, the most preferred formula, combines the lubricity and viscosity building capacity of polyethylene glycol 600 with the preferred lower molecular weight PVP grade Kollidone 30.

For use as in vivo biocompatible ultrasound couplants, the PVP gels generally must be sterile. The common and acceptable sterilization methods of high temperature autoclave and high energy sterilization using e-beam and gamma irradiation are suitable for polyvinylpyrrolidone gel formulations of the inventive device in applications that require thin pseudoplastic film formation. Radiation dosages prescribed for terminal sterilization protocols, generally 25 KGY and above do not appear to cross-link or cause chain scission of these gels. Such response is unacceptable if such high energy exposure decreases lubricity and changes flow behavior by creating insoluble solids and cohesive masses that are not easily spread into a thin film or layer between the active face of an ultrasound probe and skin, or the ionizing energy breaks the polymer bonds, thus reducing the viscosity.

In one example of manufacture, the base polymer solution is compounded in a reactor vessel suitable for vacuum degassing and heating. PVP is dissolved in glycol and/or polyol blends such as propylene glycol, glycerol, polyalkylene glycols by first vacuum degassing the slurry to remove entrapped gasses, followed by nitrogen backfill to 1 atmosphere with subsequent heating in the range of 40 to 80°C to assure complete solution. Once the polymer is completely in solution, the gel is cooled for packaging into suitable containers for sterilization in final form,

The inventive couplant fluids or gels, being in vivo biocompatible and bio-eliminated, can remain in the body without harming such since they are subsequently excreted from the body after being eroded, metabolized or absorbed via natural pathways and processes. In sterile form, the inventive in vivo biocompatible ultrasound couplants provide utility and safety for use when ultrasound examinations are performed in contact with organs, tissue and body fluids.

For use in intraoperative and intracavity procedures, the inventive couplant is generally placed inside a protective cover in contact with the ultrasound probe face to couple the acoustic energy between the active area of the probe, the ultrasound transducer, and the cover or sleeve. Since during a surgical or intracavity ultrasound examination, or therapeutic procedure, the external surface of the probe cover is in contact with body fluids that naturally conduct acoustic energy, additional couplant on the external surface of the probe cover is seldom required. In the event of accidental rupture of the protective cover, introduction of the inventive ultrasound couplant into the body cavity can result in its contact with tissue, organs and fluids. Should such an event occur, the couplants of this inventive device will not adversely affect the patient due to their biocompatibility and bio-elimination in vivo.

For patient comfort during intracavity, i.e. vaginal, rectal and transesophageal ultrasound examinations or therapeutic procedure, a lubricant such as the inventive device is often required on the exterior of the transducer protective probe cover or the endoscope shaft prior to introduction into body cavities. In instances when such in vivo biocompatible couplants are used for transcutaneous scanning or therapy, ophthalmic imaging or ultrasound guided needle punctures, such as amniocentesis and transcutaneous biopsy procedures, additional couplant is generally required to couple sound between the external surface of the protective cover or sleeve and the patient. Such couplant is usually placed on the skin of the patient in the examination area. Needle punctures made through the gel covered area of the skin can result in transport of the gel material into the underlying tissues and organs which provides the potential for undesirable immune responses should such couplants not be in vivo biocompatible .

In instances where an ultrasound probe is covered by a protective sheath, as previously mentioned, the inventive ultrasound couplants provide not only acceptable acoustic coupling properties, when such couplant is placed on the outside of the protective sheath; but, also when placed within the sheath (i.e. between the active face of the ultrasound probe and the sheath).

While the invention has been described with reference to preferred embodiments it is to be understood that the invention is not limited to the particulars thereof.

## Claims

1. An in vivo biocompatible and bio-excretable lubricant and ultrasound couplant fluid or gel comprising:
1-99 wt.% glycols, polyols and/or fats and esters thereof,
polyvinylpyrrolidone, which may be crosslinked or uncrosslinked, and
said fluid or gel containing no water.

2. The in vivo biocompatible and bio-excretable lubricant and ultrasound couplant fluid or gel of claim 1 containing:
1-99 wt.% glycols, polyols and/or fats and esters thereof,
the balance being polyvinylpyrrolidone.

3. The in vivo biocompatible and bio-excretable lubricant and ultrasound couplant fluid or gel of claim 1 wherein the polyvinylpyrrolidone is crosslinked by cross-linkers selected from at least one of chitosan derivatives, polyethyleneimine, amines and aldehydes.

4. The in vivo biocompatible and bio-excretable lubricant and ultrasound couplant fluid or gel of claim 1 or 3 containing at least one of monoglycerides and triglycerides..

5. The in vivo biocompatible and bio-excretable lubricant and ultrasound couplant fluid or gel of any one of the previous claims wherein the glycols, polyols and/or fats and esters thereof are in an amount of 10-98 wt.%.

6. The in vivo biocompatible and bio-excretable lubricant and ultrasound couplant fluid or gel of claim 5 wherein the glycols, polyols and/or fats and esters thereof are in an amount of 70-98 wt.%.

7. The in vivo biocompatible and bio-excretable lubricant and ultrasound couplant fluid or gel of any one of claims 1 to 4 wherein the polyvinylpyrrolidone is in an amount of 1-60 wt. %.

8. The in vivo biocompatible and bio-excretable lubricant and ultrasound couplant fluid or gel of any one of the previous claims wherein said glycols, polyols and/or fats and esters thereof comprise two or more carbon atoms.

9. The in vivo biocompatible and bio-excretable lubricant and ultrasound couplant fluid or gel of claim 8 wherein said glycols, polyols and/or fats and esters thereof have up to six carbon atoms.

10. The in vivo biocompatible and bio-excretable lubricant and ultrasound couplant fluid or gel of any one of the previous claims containing at least one of propylene glycol, glycerol, polyethylene glycol and sorbitol.

11. The in vivo biocompatible and bio-excretable lubricant and ultrasound couplant fluid or gel of claim 10 containing a blend of polyethylene glycol and propylene glycol.

12. The in vivo biocompatible and bio-excretable lubricant and ultrasound couplant fluid or gel of claim 2 containing:
95 wt. % polyethylene glycol, and
5 wt. % polyvinylpyrrolidone.

13. The in vivo biocompatible and bio-excretable lubricant and ultrasound couplant fluid or gel of claim 2 containing:
48 wt. % propylene glycol,
28 wt. % polyethylene glycol, and
24 wt. % polyvinylpyrrolidone.

14. The in vivo biocompatible and bio-excretable lubricant and ultrasound couplant fluid or gel of claim 2 containing:
48 wt. % propylene glycol,
30 wt. % polyethylene glycol
22 wt. % polyvinylpyrrolidone.

15. The in vivo biocompatible and bio-excretable lubricant and ultrasound couplant fluid or gel of claim 2 containing:
48 wt. % propylene glycol,
26 wt. % polyethylene glycol
26 wt. % polyvinylpyrrolidone.

16. The in vivo biocompatible and bio-excretable lubricant and ultrasound couplant fluid or gel of claim 2 containing:
70-98 wt. % of at least one of propylene glycol, glycerol, polyethylene glycol and sorbitol.

17. The in vivo biocompatible and bio-excretable lubricant and ultrasound couplant fluid or gel of any one of the previous claims wherein the fluid or gel is sterile.

18. The in vivo biocompatible and bio-excretable lubricant and ultrasound couplant fluid or gel of any one of the previous claims wherein said polyvinylpyrrolidone has a molecular weight of 2000-1,500,000 daltons.

19. The in vivo biocompatible and bio-excretable lubricant and ultrasound couplant fluid or gel of claim 18 wherein said polyvinylpyrrolidone has a molecular weight of 40,000-54,000 daltons.
